# FASCICULE DE BREVET EUROPEEN

(11) **EP 0 334 947 B1**
(45) Date de publication et mention de la délivrance du brevet: **10.03.1993**
(21) Numéro de dépôt: 88909553.5
(22) Date de dépôt: 05.10.1988
(51) Int. Cl.: G01N 33/52, G01N 33/543

(54) **DISPOSITIF DE DETECTION IMMUNOENZYMATIQUE**
IMMUNOENZYMATISCHES TESTGERÄT
IMMUNOENZYMATIC DETECTION DEVICE

(30) Priorité: 05.10.1987 FR 8713879
(43) Date de publication de la demande: 04.10.1989
(73) Titulaire: QUICK - TEST, 92532 LEVALLOIS-PERRET (FR)
(72) Inventeur: TOLEDANO, Jacques, F-75020 Paris (FR)
(74) Mandataire: Ores, Irène
(86) Numéro de dépôt international: FR8800493
(87) Numéro de publication internationale: WO8903039

(56) Documents cités:
- EP-A- 133 700
- EP-A- 0 054 679
- EP-A- 0 191 640
- EP-A- 0 200 540
- EP-A- 0 212 989
- EP-A- 0 231 830
- EP-A- 0 236 768
- WO-A-80/02077
- WO-A-86/04683
- FR-A- 2 514 511
- US-A- 4 459 358

## Description

La présente invention est relative à un dispositif de détection de la présence, dans un milieu biologique, de substances chimiques ou biologiques, même présentes à de très faibles concentrations, de l'ordre du nanogramme par millilitre. L'invention est plus particulièrement relative à des tests biologiques rapides utilisant le principe de la réaction antigène-anticorps pour révéler, par coloration d'un substrat, la présence de substances que l'on cherche à détecter.

On connaît de longue date les bandelettes de diagnostic utilisées pour effectuer des tests de détection dans les urines. On connaît également des bandelettes comportant un carré de cellulose pour le dosage de glucose à partir d'une goutte de sang déposée sur le carré de cellulose, maintenue pendant une minute sur celui-ci, puis lavée pour lire la réaction colorée.

Il existe également des dispositifs de détection immuno-enzymatique utilisant des antigènes ou des anticorps mais nécessitant, d'une part, du personnel pour prise de sang, manipulations et, d'autre part, utilisant un système complexe, coûteux et surtout très long (3 heures) en raison de lavages répétés suivant des périodes d'incubation d'une demi-heure entre chaque phase.

On connaît également, par exemple par la Demande Internationale PCT WO 86/04683, un dispositif de diagnostic immunologique formé de plusieurs zones adjacentes successives :
- une zone destinée à débarrasser le liquide à analyser des contaminants qui peuvent interférer avec le diagnostic ;
- une zone où sont adsorbés de façon réversible, des antigènes ou des anticorps conjugués à une enzyme ;
- une zone contenant des anticorps ou des antigènes immobilisés ;
- une zone de détection, contenant un substrat chromogène, de l'enzyme précitée.

Le liquide à analyser circule à travers les différentes zones en réagissant au passage avec les réactifs qui y sont incorporés.

Le Brevet Français 2 514 511 décrit également un dispositif similaire.

Bien que ces dispositifs permettent de simplifier les manipulations nécessaires aux dosages immunoenzymatiques, ils peuvent poser, toutefois, un autre problème : si la vitesse de circulation du liquide à travers les différentes zones est trop importante par rapport à la vitesse de réaction, des espèces n'ayant pas réagi (par exemple des anticorps marqués ne s'étant pas fixés sur l'antigène immobilisé de la zone suivante) risquent de parvenir à la zone de détection et de fausser les résultats.

Il est connu par la Demande de Brevet Européen N° 0 133 700 DU PONT DE NEMOURS de pourvoir dans un dispositif de test à plusieurs couches constitué par une zone réactionnelle unique, une membrane de barrage perforée dont les perforations sont remplies d'un matériau thermo-sensible qui fond par chauffage et permet la communication de fluide entre les couches fonctionnelles de la zone réactionnelle du dispositif de test.

Cependant, comme on le sait, l'intervention de températures élevées dans les tests immunologiques est de nature à provoquer la destruction ou à tout le moins l'altération des réactifs mis en oeuvre dans les tests immunologiques aussi bien que la destruction ou à tout le moins l'altération des composants dont on recherche la présence.

Le dispositif selon l'invention permet de remédier à ces inconvénients et de mettre à la disposition de patients, de médecins, de petits Centres de Soins, de Transfusion, de Services d'urgences, etc... toute une gamme de tests rapides, puisque ce dispositif, qui ne nécessite qu'une goutte de liquide à analyser, permet un diagnostic dans les quelques minutes suivantes sans aucune intervention humaine, en lisant simplement la coloration d'une pastille de substrat située à la partie inférieure du dispositif, d'où son coût réduit et sa grande facilité d'utilisation.

La présente invention a pour objet un dispositif pour la détection de la présence dans un milieu biologique liquide, de substances, même présentes à de très faibles concentrations, de l'ordre du nanogramme par millilitre, lequel dispositif est caractérisé en ce qu'il comprend, en combinaison :
- une pellicule en matière plastique opaque ou transparente (1) découpée pour former un réservoir supérieur (R1) destiné à recevoir une goutte de milieu biologique à analyser ;
- une première membrane (2) filtrante et temporairement imperméable, située à la base du réservoir (R1) et dont la face supérieure porte une mince couche d'un réactif (A) destiné à réagir, le cas échéant, avec les substances à détecter contenues dans la goutte de milieu biologique à analyser ;
- une deuxième pellicule en matière plastique opaque ou transparente (3) découpée pour former un réservoir médian (R2) destiné à recevoir le liquide provenant du passage de la goutte à travers la membrane (2), après dissolution de cette dernière par contact avec ledit liquide, lequel réservoir (R2) contient un réactif (B) propre à réagir le cas échéant avec des substances contenues dans ledit liquide ;
- une deuxième membrane (4) également filtrante et temporairement imperméable, située à la base du réservoir (R2) et à travers laquelle passe le liquide qui l'a dissoute, après son contact avec le réactif (B) dans ledit réservoir (R2) ;
- une troisième pellicule (5) en matière plastique opaque ou transparente découpée pour former un réservoir inférieur (R3) lequel contient un support approprié pour un réactif (C) qui, par contact avec le liquide qui parvient dans le réservoir (R3) après avoir traversé la membrane (4), présente une coloration propre à indiquer la présence ou l'absence de la substance recherchée dans le milieu biologique analysé.

Selon un mode de réalisation avantageux du dispositif conforme à la présente invention, le réactif (A) est un anticorps ou un antigène, de préférence lyophilisé, et marqué par un marqueur approprié ; le réactif (B) est constitué par des microbilles en matériau approprié revêtues d'un antigène ou d'un anticorps ; le réactif (C) est un substrat propre à réagir avec le marqueur du réactif (A) pour donner, le cas échéant, une coloration au contact du réactif (A).

Selon un autre mode de réalisation avantageux du dispositif conforme à la présente invention, le réactif (A) est constitué par un anticorps non marqué présent en quantité prédéterminée et le dispositif comprend, à la suite du réservoir supérieur, un réactif (D) constitué par des anticorps marqués contenus dans des liposomes et qui sont aptes à fixer l'excès de substance recherchée non fixé par la quantité prédéterminée d'anticorps non marqué précité.

Selon encore un autre mode de réalisation avantageux du dispositif conforme à la présente invention, le réactif (C) est porté par un support approprié tel que, notamment, une pastille de cellulose ou analogue.

Selon un autre mode de réalisation avantageux du dispositif conforme à la présente invention, celui-ci comprend une pluralité de réservoirs découpés dans chaque pellicule, pour permettre la détection simultanée de plusieurs substances différentes.

Selon un mode de réalisation avantageux du dispositif conforme à la présente invention, le réactif (A) est constitué par une pluralité d'anticorps ou d'antigènes différents spécifiques d'une affection et le réactif (C) comprend une pluralité de substrats propres à réagir avec un ou plusieurs des marqueurs qui constituent le réactif (A) pour donner, le cas échéant, une ou plusieurs colorations.

Selon un autre mode de réalisation avantageux du dispositif conforme à la présente invention, les membranes filtrantes sont en gélatine et fibres de verre et sont dissoutes par contact avec le milieu biologique à tester, à la température ambiante.

Outre les dispositions qui précèdent, l'invention comprend encore d'autres dispositions qui ressortiront de la description qui va suivre.

L'invention sera mieux comprise à l'aide du complément de description qui va suivre, qui se réfère au dessin annexé dans lequel :
- la Figure 1 est une vue en coupe longitudinale du dispositif conforme à l'invention ;
- la Figure 2 en est une vue en perspective ;
- la Figure 3 en est une vue de dessus, et
- la Figure 4 en est vue de dessous.

Il doit être bien entendu, toutefois, que ce dessin et les parties descriptives correspondantes sont donnés uniquement à titre d'illustration de l'objet de l'invention, dont ils ne constituent en aucune manière une limitation.

Le dispositif, représenté au dessin est constitué d'éléments superposés (1) à (5), tous de même longueur, par exemple 100 mm, et de même largeur, par exemple 20 mm, tous présentant au même endroit une ou plusieurs découpes (Réservoirs R1, R2 et R3) qui peuvent être circulaires avec un diamètre de 5 à 20 mm selon les diagnostics demandés, mais d'épaisseurs différentes, à savoir respectivement de haut en bas :
(1) une pellicule de plastique transparent ou opaque de 0,5 à 5 mm d'épaisseur, destinée à recevoir en R1 la goutte de liquide à analyser.
(2) Une membrane de gélatine est fibres de verre d'épaisseur de 0,5 à 1,5 mm et supportant à sa face supérieure une fine couche de réactif (A), destiné à réagir immédiatement avec la goutte de liquide à analyser.
(3) Une pellicule de plastique transparent ou opaque de 1 à 5 mm d'épaisseur et destinée à recevoir en R2 le liquide provenant de la filtration de la goutte à travers la membrane (2), lequel liquide va entrer en contact avec le réactif (B) dans le réservoir (R2).
(4) Une membrane identique à la précédente, filtrant le liquide après son contact avec le réactif (B).
(5) Une pellicule de plastique transparente ou opaque de 0,5 à 2 mm d'épaisseur dont la découpe forme le réservoir (R3) contenant une pastille de cellulose ou tout autre support susceptible de contenir le réactif (C). Ce dernier au contact du liquide issu de la membrane (4) présente une coloration différente selon que la substance recherchée est présente ou non dans le liquide à analyser. La lecture de la réaction se fera donc en retournant simplement le dispositif au bout de une à trois minutes selon les différentes analyses.
(6) Un film adhésif opaque de protection qui recouvre R1 au dessus et R3 en dessous.

Le réactif (A) est constitué par un anticorps ou un antigène lyophilisé, marqué par tout marqueur possible, comme les enzymes, la fluorescence ou la radioactivité, etc...

La membrane (2) est en gélatine sur fibres de verre, avec une porosité variant de 0,2 à 50 µm selon les diagnostics recherchés, et supporte sur sa face supérieure le réactif (A).

La membrane (4) est également en gélatine sur fibres de verre, de porosité comprise entre 0,2 et 50 µm.

Le réactif (B) est constitué par des microbilles métalliques de diamètre variable de 10 à 250 µm, en fer ou tout autre métal, en alliages de métaux, en polyacrylamide ou tout autre matériau, enduites d'hydroxyapatite et de Protéine A pour fixer solidement, après agitation, un antigène ou un anticorps. Elles sont ensuite mises en contact pendant une à six heures avec de l'éthanolamine pour bloquer tous les sites amine et éviter ainsi toute autre réaction chimique que celle concernant l'antigène-anticorps.

Le réactif (C) est un substrat du marqueur de réactif (A) par exemple, dans le cas où l'antigène ou l'anticorps est marque par une enzyme, si cette enzyme est une peroxydase, le substrat sera de la 3-3′ 5-5′ tétraméthylbenzidine pour une coloration bleue, ou de l'oxyphenyldiamine pour une coloration orange.

Selon une variante, il peut exister plusieurs réservoirs pour détecter plusieurs substances différentes. Par exemple, il est avantageux, pour le test de diagnostic d'une hépatite, d'associer une évaluation rapide des transaminases. De même en cas d'infarctus, l'évaluation du taux des enzymes cardiaques donne une indication très précieuse sur le pronostic et la gravité des lésions. De même également, la séro-positivité du SIDA ne suffisant pas, elle peut être complétée sur la même bandelette par un test immunologique capable d'indiquer la qualité et le nombre de lymphocytes T, et permettre ainsi le suivi des séro-positifs.

Selon une variante de réalisation des réservoirs, ceux-ci peuvent être carrés, rectangulaires, ovales, etc...

Le dispositif conforme à l'invention permet de réaliser le dépistage de nombreuses affections mettant en cause un agent (l'antigène) et la réaction de l'organisme à cet agent, c'est-à-dire la révélation et le dosage des anticorps contre cet agent à partir d'une goutte de tout liquide provenant du corps humain (sang, sérum, plasma, urines, salive, larmes, liquide céphalo-rachidien, humeurs urétrales, vaginales, lait maternel, liquides prélevés en per-opératoire ou sur des organes à greffer, pour détecter le Sida par exemple), etc...

La détection peut également se faire à partir de fragments de solides quelconques solubilisés au préalable ou simplement agités avec un peu d'eau.

Le dispositif de détection conforme à la présente invention s'applique également en médecine vétérinaire, pour le dépistage d'infections ou le dosage d'hormones par exemple.

Il s'applique également au domaine agroalimentaire chaque fois qu'il s'agit de dépister ou doser toutes substances chimiques (germes, teneur en vitamines par exemple).

L'invention s'applique aussi à l'Agriculture pour tous microdosages.

Elle s'applique également, dans un contexte plus général, à tout ce qui concerne les microdosages nécessaires pour des enquêtes, recherches, traitement des eaux, écologie diagnostic de malades chez des espèces en voie de disparition, par exemple).

Le dispositif selon l'invention est propre à doser le taux d'antigènes de certains cancers, résolvant le dramatique problème des patients traités, chez lesquels il est pratiquement impossible de savoir suffisamment tôt s'ils font des rechutes, des métastases, alors que le dispositif conforme à l'invention permet de les révéler par la réaction colorée dès que le taux d'antigènes dépasse une certaine limite.

Il est également destiné à la détection précoce et rapide de nombreuses affections (Syphilis, SIDA, Hépatites,...) et à obtenir des indications bilogiques rapides telles les groupages sanguins, l'évaluation d'enzymes circulantes et enfin le diagnostic d'affections difficiles à apprécier (Rage, Paludisme...).

On trouvera, ci-après, une liste non exhaustive des affections susceptibles d'être diagnostiquées à l'aide du dispositif conforme à la présente invention, classées comme suit:
I - Diagnostic des affections virales,
II - Diagnostic des affections microbiennes,
III - Dosages enzymatiques,
IV - Maladies auto-immunes et Maladie d'Alzheimer.
V - Groupages sanguins rapides,
VI - Dépistage de cancers.
VII - Dosage de médicaments circulants,
VIIII - Détection d'allergènes.
   Diagnostic d'allergies.
IX - Dosages des récepteurs membranaires.
X - Dosages d'hormones.
XI - Dosages divers (anions, cations de l'ionogramme, métaux, et, d'une manière générale, tous les éléments de la Classification de Mendeleieff).

Le dispositif de détection conforme à la présente invention fonctionne comme suit :
1. Une goutte de liquide est déposée dans le réservoir R1.
2. Au bout d'une minute, le sérum a réagi avec le réactif (A) et la gélatine de la membrane (2) se dissout pour laisser filtrer, à travers la fibre de verre, le sérum.
3. Ce dernier entre en contact en R2 avec les microbilles recouvertes du réactif (B). Après une minute, la seconde membrane de gélatine (4) se dissout : de nouveau filtration à travers la fibre de verre et :
4. Arrivée du serum sur le réactif (C) dans le réservoir R3 pour montrer immédiatement une coloration en fonction du résultat.

En cas de recherche d'antigène :
Réaction positive : l'antigène se lie à l'anticorps (A) en R1, traverse R2 sans être retenu par les billes et arrive en R3 pour colorer le substrat.
Réaction négative : pas d'antigène; l'anticorps marque (A) sera retenu par l'antigène des microbilles et ne colorera donc pas le substrat.

En cas de recherche d'anticorps :
Même principe sauf que (A) sera un antigene marque et que sur les microbilles est fixé un anticorps pour retenir cet antigene marqué en l'absence d'anticorps dans le sérum.

Dosages semi-quantitatifs :
En A sont fixés des anticorps non marqués fixant par exemple 10 ng/ml d'antigène. Si le sérum en contient plus, il sera plus tard fixé sur des anticorps marqués enfermés dans des liposomes qui s'ouvrent après une minute. Même principe pour le dosage semi-quantitatif des anticorps.

Le dispositif conforme à la présente invention :
- permet, de par sa structure, d'éviter les différentes étapes de lavage habituelles en immunologie et donc de gagner un temps important sur les autres tests ;
- permet d'avoir des informations biologiques de première importance à partir d'une goutte de sang prélevée à un doigt, et donc de pouvoir l'utiliser en l'absence de personnel infirmier spécialisé et en l'absence de laboratoire ;
- permet une grande souplesse d'utilisation puisque le même test peut détecter une substance dans une goutte d'urine ou dans une goutte de lait ou dans un solide préalablement solubilisé.
- permet de détecter plusieurs substances différentes avec la même goutte de liquide en associant simplement dans les réservoirs décrits plus haut, les réactifs correspondant auxdites substances. Si le test est positif, c'est qu'au moins une des substances recherchée est présente dans la goutte examinée. C'est ainsi, par exemple, que l'on peut mettre dans le même test différents marqueurs de cancers (digestifs par exemple : ACE pour le colon, C19-9 pour le pancréas, etc...). Comme il est improbable que le sujet ait plusieurs cancers différents, si le test est positif, il permet de diriger le médecin vers la sphère digestive et faire, à ce moment là, des tests séparés pour chaque cancer et chaque organe pour localiser ainsi une tumeur débutante.

De même, avant une intervention chirurgicale, sur la table d'opération, le chirurgien ou l'anesthésiste peut utiliser un test dont les microbilles du réactif (B) seront recouvertes soit d'antigènes différents (HIV, HTLV, Hépatite, etc...), soit un test avec un pourcentage de billes marquées avec le HIV, une autre partie avec le HTLV, etc..., en sorte qu'une goutte de sang, dans un seul test avec le même dispositif, peut donner des indications de la plus haute importance.

## Revendications

1. Dispositif pour la détection de la présence dans un milieu biologique liquide, de substances, même présentes à de très faibles concentrations, de l'ordre du nanogramme par millilitre, lequel dispositif est caractérisé en ce qu'il comprend, en combinaison :
- une pellicule en matière plastique opaque ou transparente (1) découpée pour former un réservoir supérieur (R1) destiné à recevoir une goutte de milieu biologique à analyser ;
- une première membrane (2) filtrante et temporairement imperméable, située à la base du réservoir (R1) et dont la face supérieure porte une mince couche d'un réactif (A) destiné à réagir, le cas échéant, avec les substances à détecter contenues dans la goutte de milieu biologique à analyser ;
- une deuxième pellicule en matière plastique opaque ou transparente (3) découpée pour former un réservoir médian (R2) destiné à recevoir le liquide provenant du passage de la goutte à travers la membrane (2) après dissolution de cette dernière par contact avec ledit liquide, lequel réservoir (R2) contient un réactif (B) propre à réagir le cas échéant avec des substances contenues dans ledit liquide ;
- une deuxième membrane (4) également filtrante et temporairement imperméable, située à la base du réservoir (R2) et à travers laquelle passe le liquide qui l'a dissoute après son contact avec le réactif (B) dans ledit réservoir (R2) ;
- une troisième pellicule (5) en matière plastique opaque ou transparente découpée pour former un réservoir inférieur (R3) lequel contient un support approprié pour un réactif (C) qui, par contact avec le liquide qui parvient dans le réservoir (R3) après avoir traversé la membrane (4), présente une coloration propre à indiquer la présence ou l'absence de la substance recherchée dans le milieu biologique analysé.

2. Dispositif selon la revendication 1, caractérisé en ce que le réactif (A) est un anticorps ou un antigène, de préférence lyophilisé, et marqué par un marqueur approprié ; le réactif (B) est constitué par des microbilles en matériau approprié revêtues d'un antigène ou d'un anticorps ; le réactif (C) est un substrat propre à réagir avec le marqueur du réactif (A) pour donner, le cas échéant, une coloration au contact du réactif (A).

3. Dispositif selon la revendication 1, caractérisé en ce que le réactif (A) est constitué par un anticorps non marqué présent en quantité prédéterminée et en ce que le dispositif comprend, à la suite du réservoir (R1), un réactif (D) constitué par des anticorps marqués contenus dans des liposomes et qui sont aptes à fixer l'excès de substance recherchée non fixé par la quantité prédéterminée d'anticorps non marqué précité.

4. Dispositif selon l'une quelconque des revendications 1 à 3, caractérisé en ce que le réactif (C) est porté par un support approprié tel que, notamment, une pastille de cellulose ou analogue.

5. Dispositif selon l'une quelconque des revendications 1 à 4, caractérisé en ce qu'il comprend une pluralité de réservoirs découpés dans chaque pellicule, pour permettre la détection simultanée de plusieurs substances différentes.

6. Dispositif selon la revendication 5, caractérisé en ce que le réactif (A) est constitué par une pluralité d'anticorps ou d'antigènes différents spécifiques d'une affection et le réactif (C) comprend une pluralité de substrats propres à réagir avec un ou plusieurs des marqueurs qui constituent le réactif (A) pour donner, le cas échéant, une ou plusieurs colorations.

7. Dispositif selon l'une quelconque des revendications 1 à 6, caractérisé en ce que les membranes filtrantes sont en gélatine et fibres de verre et sont dissoutes par contact avec le milieu biologique à tester, à la température ambiante.

## Patentansprüche

1. Testgerät um das Vorhandensein von Substanzen sogar in sehr geringen Konzentrationen in der Größenordnung von 1 Nanogramm je Milliliter in einem flüssigen biologischen Medium nachzuweisen, dadurch **gekennzeichnet,** daß es in Kombination miteinander umfaßt:
- eine Folie (1) aus opakem oder transparentem Kunststoff, ausgeschnitten, um einen oberen Behälter (R1) zu bilden, der dazu bestimmt ist, einen Tropfen des biologischen Mediums, das analysiert werden soll, aufzunehmen;
- eine erste filtrierende und zeitweilig undurchlässige Membran (2) am Boden des Behälters (R1), deren Oberseite eine dünne Schicht eines Reagens (A) trägt, das dazu bestimmt ist, im gegebenen Fall mit den Substanzen, die nachgewiesen werden sollen und die in dem Tropfen des biologischen Mediums, das analysiert werden soll, enthalten sind, zu reagieren;
- eine zweite Folie (3) aus opakem oder transparentem Kunststoff, ausgeschnitten, um einen mittleren Behälter (R2) zu bilden, dazu bestimmt, die Flüssigkeit aufzunehmen, die aus dem Durchgang des Tropfens durch die Membran (2) nach Auflösen der letzteren durch Kontakt mit der Flüssigkeit stammt, wobei der Behälter (R2) ein Reagens (B) enthält, das im gegebenen Fall mit den in der Flüssigkeit enthaltenen Substanzen reagiert;
- eine zweite, ebenfalls filtrierende und zeitweilig undurchlässige Membran (4) am Boden des Behälters (R2), und durch die die Flüssigkeit tritt, die sie, nach ihren Kontakt mit dem Reagens (B) in dem Behälter (R2) aufgelöst hat ;
- eine dritte Folie (5) aus opakem oder transparentem Kunststoff, ausgeschnitten, um einen unteren Behälter (R3) zu bilden, der einen geeigneten Träger für ein Reagens (C) enthält, welches bei Kontakt mit der Flüssigkeit, die in den Behälter (R3) eintritt, nachdem sie die Membran (4) passiert hat, eine geeignete Färbung aufweist, um das Vorhandensein oder die Abwesenheit der in dem analysierten biologischen Medium gesuchten Substanz anzuzeigen.

2. Testgerät nach Anspruch 1, dadurch **gekennzeichnet,** daß das Reagens (A) ein Antikörper oder ein Antigen ist, vorzugsweise lyophilisiert, und mit einem geeigneten Marker markiert; daß das Reagens (B) aus Mikrokügelchen aus geeignetem Material besteht, die mit einem Antigen oder einem Antikörper überzogen sind; daß das Reagens (C) ein Substrat ist, das mit dem Marker des Reagens (A) reagieren kann, um im gegebenen Fall bei Kontakt mit dem Reagens (A) eine Färbung zu ergeben.

3. Testgerät nach Anspruch 1, dadurch **gekennzeichnet,** daß das Reagens (A) aus einem nicht-markierten Antikörper besteht, der in vorbestimmter Menge vorhanden ist, und daß die Vorrichtung anschließend an den Behälter (R1) ein Reagens (D) umfaßt, das aus markierten Antikörpern besteht, die in Liposomen enthalten sind und den Überschuß der gesuchten, von der vorgegebenen Menge nichtmarkierter Antikörper nicht gebundenen Substanz binden können.

4. Testgerät nach einem der Ansprüche 1 bis 3, dadurch **gekennzeichnet,** daß das Reagens (C) auf einem geeigneten Träger, wie vor allem einer vorgeformten Tablette aus Cellulose oder einem analogen Träger, getragen wird.

5. Testgerät nach einem der Ansprüche 1 bis 4, dadurch **gekennzeichnet,** daß es mehrere, in jeder Folie ausgeschnittene Behälter umfaßt, um den gleichzeitigen Nachweis von mehreren unterschiedlichen Substanzen zu ermöglichen.

6. Testgerät nach Anspruch 5, dadurch **gekennzeichnet,** daß das Reagens (A) aus mehreren verschiedenen Antikörpern oder Antigenen, die für eine Erkrankung spezifisch sind, besteht und das Reagens (C) mehrere Substrate umfaßt, die mit einem oder mehreren der Marker, aus denen das Reagens (A) besteht, reagieren können, um im gegebenen Fall eine oder mehrere Färbungen zu ergeben.

7. Testgerät nach einem der Ansprüche 1 bis 6, dadurch **gekennzeichnet,** daß die Filtermembranen aus Gelatine und Glasfasern bestehen und bei Kontakt mit dem biologischen Medium, das untersucht werden soll, bei Raumtemperatur aufgelöst werden.

## Claims

1. A device for the detection of the presence in a liquid biological medium of substances even present in very low concentrations, of the order of one manogram per millilitre said device being characterized in that it comprises in combination :
- an opaque or transparent plastics film (1) cut away to form an upper reservoir (R1) adapted to receive a droplet of the biological medium to be analysed ;
- a first, temporarily impermeable filter diaphragm which is disposed at the base of the reservoir (R1) and whose upper face bears a thin layer of a reagent (A) adapted to react with any substances to be detected contained in the droplet of the biological medium to be analysed ;
- a second opaque or transparent plastics film cut away to form a median reservoir (R2) adapted to receive the liquid coming from the flow of the droplet through the diaphragm (2) after the dissolution thereof by contact with said liquid, said reservoir (R2) containing a reagent (B) adapted to react with any substances contained in said liquid ;
- a second temporarily impermeable filter diaphragm (4) which is disposed at the base of the reservoir (R2) and through which the liquid flows which has dissolved said diaphragm after its contact with the reagent (B) in said reservoir (R2) ;
- a third opaque or transparent plastics film cut away to form a lower reservoir (R3) containing a suitable support for a reagent (C) which, by contact with the liquid which arrives in the reservoir (R3) after passing through the diaphragm (4), has a colouring adapted to indicate the presence or absence of the substance looked for in the biological medium analysed.

2. A device according to claim 1, characterized in that !he reagent (A) is an antibody or an antigen preferably lyophilised, labelled with a suitable label ; the reagent (B) is formed by microspheres of a suitable material coated with an antigen or an antibody ; the reagent (C) is a substrate adapted to react with the label of the reagent (A) to possibly give a colouring in contact with the reagent (A).

3. A device according to claim 1, characterized in that the reagent (A) is formed by an unlabelled antibody present in a predetermined quantity and the device comprises, following the reservoir (R1), a reagent (D) formed by labelled antibodies which are contained in liposomes and which are adapted to bind the excess of the looked for substance which has not been bound by the predetermined quantity of the aforementioned unlabelled antibody.

4. A device according to any of claims 1 to 3, characterized in that the reagent (C) is borne by a suitable support such as more particularly a cellulose pellet or the like.

5. A device according to any of claims 1 to 4, characterized in that it comprises a plurality of reservoirs cut out in each film to allow the simultaneous detection of a number of different substances.

6. A device according to claim 5, characterized in that the reagent (A) is formed by a plurality of different antibodies or antigens specific to a disease and the reagent (C) comprises a plurality of substrates adapted to react with one or more of the labels forming the reagent (A) to possibly give one or more colourings.

7. A device according to any of claims 1 to 6, characterized in that the filter diaphragms are made of gelatine and glass fibres and are dissolved by contact with the medium to be tested at ambient temperature.
